# EUROPEAN PATENT SPECIFICATION

(11) **EP 1 933 835 B1**
(45) Date of publication and mention of the grant of the patent: **08.12.2010**
(21) Application number: 06819072.7
(22) Date of filing: 04.10.2006
(51) Int. Cl.: A61K 31/429, A61P 29/00, C07D 513/04

(54) **TETRAHYDRO-PYRROLO[1,2-B]ISOTHIAZOLE 1,1-DIOXIDES AS LFA-1 INHIBITORS**
TETRAHYDRO-PYRROLO[1,2-B]ISOTHIAZOL 1,1-DIOXIDE ALS LFA-1 INHIBITOREN
TÉTRAHYDRO-PYRROLO[1,2-B]ISOTHIAZOLE-1,1-DIOXYDES EN TANT QU'INHIBITEURS DE LFA-1

(30) Priority: 06.10.2005 GB 0520378
(43) Date of publication of application: 25.06.2008
(73) Proprietor: Novartis AG, 4056 Basel (CH)
(72) Inventor: BAUMANN, Karl, A-1235 Vienna (AT)
(74) Representative: de Weerd, Petrus G.W.
(86) International application number: PCT/EP2006/067017
(87) International publication number: WO 2007/039616

(56) References cited:
- WO-A-99/11258
- WO-A2-2005/089118
- HANESSIAN S ET AL: "Synthesis of functionally diverse bicyclic sulfonamides as constrained proline analogues and application to the design of potential thrombin inhibitors" TETRAHEDRON, ELSEVIER SCIENCE PUBLISHERS, AMSTERDAM, NL, vol. 59, no. 35, 25 August 2003 (2003-08-25), pages 7047-7056, XP004448510 ISSN: 0040-4020
- COOPER G F: "SYNTHESIS OF SUBSTITUTED 1,3-PROPANESULTAMS FROM N-SUBSTITUTED 2-AMINO ALCOHOLS" SYNTHESIS, GEORG THIEME VERLAG, STUTTGART, DE, vol. 10, October 1991 (1991-10), pages 859-860, XP001118214 ISSN: 0039-7881
- YUSUF-MAKAGIANSAR H ET AL: "INHIBITION OF LFA-1/ICAM-1 AND VLA-4/VCAM-1 AS A THERAPEUTIC APPROACH TO INFLAMMATION AND AUTOIMMUNE DISEASES" MEDICINAL RESEARCH REVIEWS, NEW YORK, NY, US, vol. 22, no. 2, March 2002 (2002-03), pages 146-167, XP009034558 ISSN: 0198-6325
- CLARK D A ET AL: "Castanospermine, an oligosaccharide processing inhibitor, reduces both lymphocyte-endothelial cell binding and LFA-1alpha membrane expression" TRANSPLANTATION PROCEEDINGS, vol. 33, no. 1-2, February 2001 (2001-02), page 522, XP002409063 & XVIII INTERNATIONAL CONGRESS OF THE TRANSPLANTATION SOCIETY; ROME, ITALY; AUGUST 29-SEPTEMBER 01, 2000 ISSN: 0041-1345

## Description

The present invention relates to tetrahydro-pyrrolo[1,2-b]isothiazole 1,1-dioxides which are pharmaceutically active, e.g. for use in the treatment of disorders mediated by interactions of LFA-1 with its ligands involved in cell adhesion, migration and activation.

Inflammatory recruitment of leukocytes is governed by dynamic interactions between integrins and endothelial immunoglobulin superfamily (IgSF) proteins, such as ICAM-1 (intercellular adhesion molecule-1), ICAM-2, ICAM-3 and the IgSF member junctional adhesion molecule 1 (JAM-1), all of which have been identified to be a ligand of the beta(2) integrin lymphocyte function-associated antigen 1 (LFA-1). Compounds which mediate, e.g. inhibit, interaction of LFA-1 and its ligands involved in cell adhesion, migration and activation have been found to constitute a therapeutic approach to inflammation and autoimmune diseases.

WO 2005/089118 describes bicyclic compounds for use in the treatment of androgen receptor-associated diseases, not specifically disclosing 3a,4,5,6-tetrahydro-pyrrolo[1,2-b]iso-thiazole dioxides for a medical use. S. Hanessian et al., Tetrahedron 59(2003), 7047-56, and G.F. Cooper et al., Synthesis 10(1991), 859-60, disclose the three compounds disclaimed in present claim 1 as such without providing a pharmaceutical use for them.

Now surprisingly it was found that 3a,4,5,6-tetrahydro-pyrrolo[1,2-b]-isothiazoles wherein the sulphur is in the form of a dioxide mediate, e.g. inhibit, the activity of LFA-1 with its ligands involved in cell adhesion, migration and activation.

In one aspect the present invention, provides the use of 3a,4,5,6-tetrahydro-pyrrolo[1,2-b]-isothiazoles wherein the sulphur is in the form of a dioxide which is a compound of formula I, I_{PREF} for the preparation of a medicament for use in the treatment or prevention of disorders, including of diseases, mediated by interactions of LFA-1 with its ligands involved in cell adhesion, migration and activation
wherein 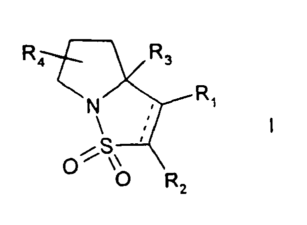 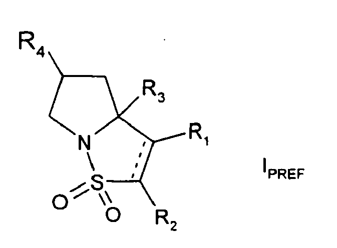 the dotted line is a bond or is no bond,
R₁ is hydrogen, optionally substituted alkyl, alkenyl, alkynyl, cycloalkyl, alkoxy, aryl or heterocyclyl, hydroxy, SH, SR₅, cyano, halogen or amino, or
the dotted line is no bond and R₁ is attached to the ring system via a double bond and is oxo, R₂ is hydrogen or optionally substituted cycloalkyl, aryl, or heterocyclyl,
R₃ is hydrogen, COOR₆, aminocarbonyl, or optionally substituted alkyl, alkenyl, alkynyl, aralkyl, alkoxy, cycloalkyloxy, aryloxy, or heterocycyloxy,
R₄ is hydrogen, halogen, hydroxy, SH, optionally substituted alkyl, alkenyl, alkynyl, alkoxy or alkylthio, or a silyl group such as trialkylsilyl or trialkylsilyloxy, e.g. tri(C₁₋₆)alkylsilyl(oxy), N₃, amino, or
R₄ is heterocyclyl comprising at least one nitrogen atom as a heteroatom and being bound via that nitrogen atom to a compound of formula I, or
R₄ is attached to the ring system by a double bond and is oxo; and
R₅ and R₆ independently of each other are alkyl, alkenyl, alkynyl, cycloalkyl, aryl or heterocyclyl.

In another aspect the present invention provides a 3a,4,5,6-tetrahydro-pyrrolo[1,2-b]-isothiazole wherein the sulphur is in the form of a dioxide which is a compound of formula 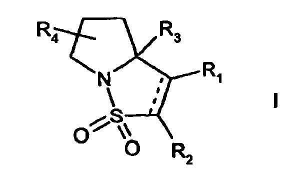 such as a compound of formula 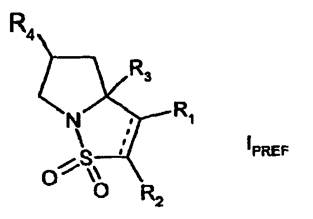 wherein
the dotted line is a bond or is no bond,
R₁ is hydrogen, optionally substituted alkyl, alkenyl, alkynyl, cycloalkyl, alkoxy, aryl or heterocyclyl, hydroxy, SH, SR₅, cyano, halogen or amino, or
the dotted line is no bond and R₁ is attached to the ring system via a double bond and is oxo,
R₂ is hydrogen or optionally substituted cycloalkyl, aryl, or heterocyclyl,
R₃ is hydrogen, COOR₆, aminocarbonyl, or optionally substituted alkyl, alkenyl, alkynyl, aralkyl, alkoxy, cycloalkyloxy, aryloxy, or heterocycyloxy,
R₄ is hydrogen, halogen, hydroxy, SH, optionally substituted alkyl, alkenyl, alkynyl, alkoxy or alkylthio, or a silyl group such as trialkylsilyl or trialkylsilyloxy, e.g. tri(C₁₋₆)alkylsilyl(oxy), N₃, amino, or
R₄ is heterocyclyl comprising at least one nitrogen atom as a heteroatom and being bound via that nitrogen atom to a compound of formula I, or
R₄ is attached to the ring system by a double bond and is oxo; and
R₅ and R₆ independently of each other are alkyl, alkenyl, alkynyl, cycloalkyl, aryl or heterocyclyl,
with the exception of (6S)-6-(tert-butyl-diphenyl-silanyloxymethyl)-3a,4,5,6-tetrahydro-pyrrolo[1,2-b]isothiazole 1,1-dioxide, (6S)-(1,1-dioxo-3a,4,5,6-tetrahydro-1H-λ⁶-pyrrolo[1,2-b]isothiazol-6-yl)-methanol and (3aS)-2,3,3a,4,5,6-hexahydropyrrolo[1,2-b]isothiazole 1,1-dioxide.

In a compound of formula I **preferably** the dotted line is a bond, or is no bond, preferably the dotted line is a bond, and R₁ is hydroxy, amino, (C₁₋₈)alkyl, (C₂₋₈)alkenyl, (C₂₋₈)alkynyl, (C₁₋₈)-alkoxy, e.g. including (C₁₋₈)alkyl, (C₂₋₈)alkenyl or (C₁₋₈)alkoxy substituted by (C₆₋₁₈)aryl, such as phenyl; or the dotted line is no bond and R₁ is attached to the ring system via a double bond and is oxo, e.g. the dotted line is a bond and R₁ is hydroxy or (C₁₋₈)alkoxy, or the dotted line is a single bond and R₁ is attached to the ring system via a double bond and is oxo.

In a compound of formula I **preferably** R₂ is hydrogen, unsubstituted aryl, or substituted aryl, such as halophenyl, e.g. including dihalophenyl, such as 3,5-dihalophenyl, e.g. 3,5-dichlorophenyl, or hydrogen.

In a compound of formula I **preferably** R₃ is optionally substituted alkyl, alkenyl, or alkynyl, e.g. of up to 8 carbon atoms, preferably substituted by aryl, such as substituted by substituted aryl, e.g. including (C₆₋₁₈)aryl(C₁₋₄)alkyl, e.g. phenyl(C₁₋₄)alkyl; e.g. optionally substituted phenylmethyl, such as phenylmethyl, halophenylmethyl, e.g. bromophenylmethyl, cyanophenylmethyl, such as halophenylmethyl.

In a compound of formula I **preferably** R₄ is hydroxy, a silyl group, such as tri(C₁₋₆)alkylsilyloxy, e.g. (tert.butyl)(dimethyl)silyloxy, alkoxy, such as (C₁₋₄)alkoxy, amino, or R₄ is heterocyclyl comprising at least one nitrogen atom as a heteroatom and being bound via that nitrogen atom to a compound of formula I, or
R₄ is attached to the ring system by a double bond and is oxo;
such as hyxdroxy, (C₁₋₄)alkoxy, tri(C₁₋₆)alkylsilyloxy or oxo.

In another aspect the present invention provides a compound of formula I, such as a compound of formula I_{PREF}
wherein
- R₁ is (C₁₋₆)alkoxy, (e.g., where the dotted line is a bond, methoxy), or R₁ is attached to the ring system via a double bond and is oxo,
- R₂ is substituted phenyl (such as, where the dotted line is a bond, phenyl substituted by halogen, such as 3,5-dihalophenyl, e.g. 3,5-dichlorophenyl),
- R₃ is substituted phenylmethyl (e.g. , where the dotted line is a bond, wherein phenyl is substituted by halogen, such as halobenzyl, e.g. 4-bromophenylmethyl), and
- R₄ is hydroxy, trialkylsilyloxy (such as, where the dotted line is a bond, (C₁₋₆)trialkylsilyloxy), or R₄ is attached via a double bond and is oxo.

The meanings of the residues in a compound provided by the present invention as defined herein include the following meanings:
Alkyl as a substituent or part of a substituent includes (C₁₋₂₀)alkyl, such as (C₁₋₈)alkyl, e.g. (C₁₋₄)alkyl. Alkenyl as a substituent or part of a substituent includes (C₂₋₂₀)alkenyl, such as (C₂₋₈)alkenyl. Alkynyl as a substituent or part of a substituent includes (C₂₋₂₀)alkynyl, such as (C₂₋₈)alkynyl Cycloalkyl as a substituent or part of a substituent includes saturated or partially unsaturated cycloalkyl, such as (C₃₋₁₂)cycloalkyl.
Aryl as a substituent or part of a substituent includes (C₆₋₁₈)aryl, such as phenyl, naphthyl. Heterocyclyl as a substituent or part of a substituent includes aliphatic heterocyclyl and aromatic heterocyclyl, having 3 to 8 ring members and 1 to 4 hetroatoms selected from N, O, S. Acyl incudes alkylcarbonyl, cycloalkylcyrbonyl, arylcarbonyl or heterocyclylcarbonyl. Alkoxy includes (C₁₋₂₀)alkoxy, such as (C₁₋₄)alkoxy.
Amino includes unsubstituted and substituted amino, e.g. including
   alkylamino, such as (C₁₋₈)alkylamino, dialkylamino, such as (C₁₋₈)dialkylamino, cycloalkylamino, such as (C₃₋₁₂)cycloalkylamino, arylamino, e.g. including (C₆₋₁₂)arylamino, acylamino, e.g. including (C₂₋₁₈)acylamino, such as (C₁₋₈)alkylcarbonylamino, (C₆₋₁₂)arylcarbonylamino, (C₃₋₁₂)cycloalkylcarbonylamino, and heterocyclylcarbonylamino; (acyl)(alkyl)-amino, such as ((C₂₋₁₈)acyl)-((C₁₋₄)alkyl))-amino, e.g. alkyl N-methyl-N-methylcarbonyl-amino, N-benzyl-N-methylcarbonyl-amino, N-ethyl-N-methylcarbonyl-amino, sulfonylamino, such as (C₁₋₄)alkysulfonylamino, (C₆₋₁₂)arylsulfonylamino, (C₃₋₈)cyclohexylsulfonylamino, heterocycylsulfonylamino.
   Substituted alkyl, alkenyl, alkynyl, alkoxy or alkylthio includes alkyl, alkenyl, alkynyl, alkoxy or alkylthio substituted by one or more halogen, hydroxy, SH, cyano, alkylthio, heterocyclyl, aryl, carboxyl, acyl, nitro, amino, alkoxy, carboxy, amido, or a silyl group, such as triakylsilyl or trialkylsilyloxy.
   Substituted cycloalkyl, aryl or heterocyclyl or substituted cyclalkyloxy, heterocyclyloxy or aryloxy e.g. includes cycloalkyl, aryl or heterocyclyl, or cyclalkyloxy, heterocyclyloxy or aryloxy substituted by one or more
   alkyl, e.g. including haloalkyl, e.g. trifluoromethyl, cycloalkylalkyl, heterocyclyllalkyl, aralkyl, aryloxyalkyl, arylthioalkyl, arylsulfinylalkyl,
   alkenyl, such as arylalkenyl, heterocyclylalkenyl,
   alkynyl, cycloalkyl, heterocyclyl,
   aryl, e.g. including aminocarbonylaryl,
   oxo, hydroxy, SH, alkoxy, such as haloalkoxy, e.g. trifluoromethoxy, aryloxy, heterocyclyloxy, arylalkoxy, alkylthio, arylthio, heterocyclylthio, alkoxyarylthio, arylsulfinyl,
   alkylcarbonyloxy, arylcarbonyloxy, heterocyclylcarbonyloxy,
   alkylcarbonyl, alkoxycarbonyl, arylcarbonyl, aryloxycarbonyl, heterocyclylcarbonyl, heterocyclyloxycarbonyl, aminocarbonyl, e.g. including alkylaminocarbonyl, arylaminocarbonyl, sulfonaminocarbonyl, e.g. arylsulfonaminocarbonyl, halogen, nitro, cyano, amino, e.g. including alkylcarbonylamino, arylcarbonylamino, arylsulfonylamino; or triakylsilyl or trialkylsilyloxy.
Cycloalkyl, aryl and heterocyclyl substituents preferably include oxo, hydroxy, halogen, alkoxy, e.g. including (C₁₋₄)alkoxy, N₃, cyano, haloalkyl, e.g. including halo(C₁₋₄)alkyl, heterocyclyl comprising 5 or 6 ring members and 1 to 4 heteroatoms selected from N, O, S, or optionally substituted amino.

Any ring defined herein, e.g. cycloalkyl, aryl or heterocyclyl, includes a single ring and a ring fused with another ring (system).

In another aspect the present invention provides a compound of anyone of Example 1 to Example 5.

The present invention includes a compound of formula I, wherein one, more or all of the residues defined have a preferred meaning as defined above, and the other residues have a meaning as defined above.

In a compound provided by the present invention each single defined substituent may be a preferred substituent, independently of the other substituents defined.

Compounds provided by the present invention are hereinafter designated as "compound(s) of (according to) the present invention", which in the case of second medical use, or where it is merely stated that the present "disclosure describes" subsequent subject matter, also includes the compounds disclaimed in claim 1. 3a,4,5,6-Tetrahydro-pyrrolo[1,2-b]-isothiazoles wherein the sulphur is in the form of a dioxide, include a compound of formula I and I_{PREF}. A compound of the present invention includes a compound in any form, e.g. in free form, in the form of a salt, in the form of a solvate and in the form of a salt and a solvate.

In another aspect the present invention provides a compound of the present invention in the form of a salt.

Such salts include preferably pharmaceutically acceptable salts, although pharmaceutically unacceptable salts are included, e.g. for preparation / isolation / purification purposes.

A salt of a compound of the present invention includes a metal salt or an acid addition salt. A compound of the present invention in free form may be converted into a corresponding compound in the form of a salt; and vice versa. A compound of the present invention in free form or in the form of a salt and in the form of a solvate may be converted into a corresponding compound in free form or in the form of a salt in non-solvated form; and vice versa.

A compound of the present invention and an intermediate of the present invention, may exist in the form of isomers and mixtures thereof; e.g. optical isomers, diastereoisomers, cis/trans conformers. A compound of the present invention and an intermediate of the present invention may e.g. contain asymmetric carbon atoms and may thus exist in the form of enatiomers or diastereoisomers and mixtures thereof, e.g. racemates. A compound of the present invention and an intermediate of the present invention may be present in the (R)-, (S)- or (R,S)-configuration preferably in the (R)- or (S)-configuration regarding substituents in specified positions in a compound of the present invention.

For example, a carbon atom in the pyrrolidine part of the ring system of a compound of the present invention, e.g. a compound of formula I, becomes an asymmetric carbon atom if a substitutent, other than hydrogen, is attached; and a compound of the present invention correspondingly substituted may be present in the (R)-, (S)- or (R,S)-configuration, preferably in the (R)- or (S)-configuration, regarding each of the substituents at such asymmetric carbon atom in a compound of the present invention, e.g. a compound of the present invention may be present in the (R)-, (S)- or (R,S)-configuratio, preferably in the (R)- or (S)-configuration regarding R₃, or R₄.

Isomeric mixtures may be separated as appropriate, e.g. according, e.g. analogously, to a method as conventional, to obtain pure isomers. The present invention includes a compound of the present invention in any isomeric form and in any isomeric mixture.

The present invention also includes tautomers of a compound of the present invention, where tautomers can exist.

In another aspect the present disclosure describes a process for the production of a compound of formula I, comprising the steps
a. reacting a compound of formula 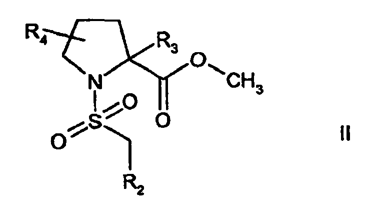 wherein R₂, R₃ and R₄ are as defined above with potassium hexamethyl disilazane, e.g. in organic solvent,
b) isolating a compound of formula I, wherein R₂, R₃ and R₄ are as defined above, and wherein R₁ is hydroxy from the reaction mixture, and optionally
c) further reacting a compound obtained in step b) to obtain a compound of formula I, wherein R₁, R₂, R₃ and R₄ are as defined above.

Further reaction e.g. includes replacing R₁ and/or R₄ in a compound of formula I obtained, to obtain a compound of formula I wherein R₁ and/or R₄ are different, e.g. by a method as appropriate, e.g. according, e.g. analogously, to a method as conventional.

A compound of formula I thus obtained may be converted into another compound of formula I, e.g. or a compound of formula I obtained in free form may be converted into a salt of a compound of formula I and vice versa.

A compound of formula II may be e.g. obtained by reacting a compound of formula 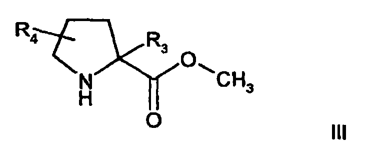 wherein R₃ and R₄ are as defined above, with a compound of formula 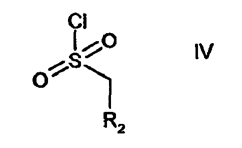 wherein R₂ is as defined above in organic solvent in the presence of a base, e.g. potassium hydrogen carbonate.

A compound of formula III may be e.g. obtained by splitting off the N-protection group in a compound of formula 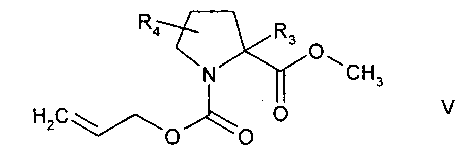 wherein R₃ and R₄ are as defined above, e.g. reacting a compound of formula V with tetrakis-(triphenylphosphine)-palladium in organic solvent in the presence of 1,4-diaza[2,2,2]bicyclooctane.

A compound of formula V may be e.g. obtained, if R₄ in a compound of formula I is other than N₃ or oxo, by reacting a compound of formula 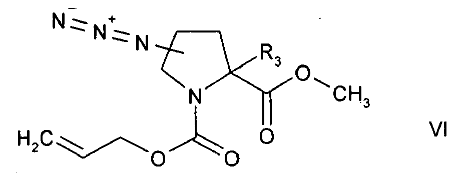 wherein R₃ is as defined above, with zinc powder in glacial acetic acid, to obtain a compound of formula 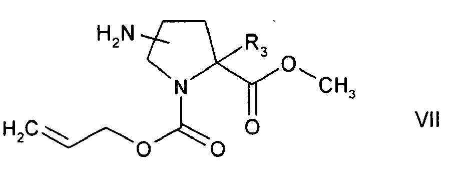 and optionally further reacting the amine group obtained, to obtain a compound of formula V, wherein R₃ and R₄ are as defined above, with the exception of R₄ is oxo.

A compound of formula VI, wherein R₃ is other than hydrogen, may be e.g. obtained by treating a compound of formula 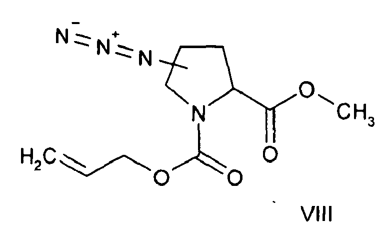 with lithium-bis-trimetylsilylamide in organic solvent and treating the mixture obtained with a compound of formula

R₃-Hal IX

wherein R₃ is as defined above with the exception of hydrogen, and Hal is halogen, such as Br.

In any intermediate of formulae II, III, IV, V, VI, VII, VIII or IX, functional groups, if present, optionally may be in protected form or in the form of a salt, if a salt-forming group is present. Protecting groups, optionally present, may be removed at an appropriate stage, e.g. according, e.g. analogously, to a method as conventional

Any compound described herein, e.g. a compound of the present invention and intermediates (starting materials) of formulae II, III, IV, V, VI, VII, VIII or IX may be prepared as appropriate, e.g. according, e.g. analogously, to a method as conventional, e.g. or as specified herein.

The compounds of the present invention exhibit valuable pharmacological properties, e.g. by mediating, such as inhibiting the activity of LFA-1 interactions with its ligands, e.g. inhibiting the activity of LFA-1/ICAM-1, LFA-1/ICAM-2, LFA-1/ICAM-3 and/or LFA-1/JAM-1 interactions, e.g. LFA-1/ICAM-1 interaction, and thus mediating, e.g. inhibiting inflammation, e.g. as indicated in vitro and in vivo TEST SYSTEMS herein. The compounds of the present invention are therefore indicated for therapy.

### A. In vitro TEST SYSTEM (Cell free assay)

The assay determines the binding of soluble human ICAM-1 to immobilized human LFA-1. LFA-1 is purified from JY cells, a human lymphoblastoid B cell-line, by immunoaffinity chromatography analogously as described by Dustin et al., J. Immunol, 148, 2654-2663, 1992. ICAM-1 mouse Cκ fusion protein (ICAM-1) is produced using the baculovirus system as described by Weitz-Schmidt et al., Anal. Biochem. 238,184-190, 1996.

Purified LFA-1 is diluted 1:20 in phosphate buffered saline (PBS) containing 2 mM MgCl₂, pH 7.4 and coated onto microtiter plates (Nunc) at 37° for 3 hours. Plates are blocked with 1% heat-treated bovine serum albumin in PBS for 2 hours at 37° followed by a washing step using PBS, 2 mM MgCl₂, 1% fetal calf serum, pH 7.4 (assay buffer). Compounds of the present invention (10 mM solution in DMSO) are diluted in assay buffer and added to the plates. Biotinylated recombinant ICAM-1 in assay buffer (6 µg/ml) is added and allowed to bind at 37° for one hour. After incubation, wells are washed with assay buffer. Streptavidin-peroxidase diluted 1:5000 in assay buffer is added and incubated for 45 min at 37°. Plates are washed with assay buffer and 2,2'-azino-bis(3-ethylbenzothiazoline-6-sulfonic acid) diammonium salt substrate solution is added to each well. The reaction is stopped after 20 minutes and bound ICAM-1 is determined by measuring the optical density at 405 nm in a microplate reader.

In this assay the compounds of the present invention exhibit activity, e.g. the compounds of the present invention inhibit adhesion of LFA-1 to ICAM.

### B. In vivo TEST SYSTEM Allergic Contact Dermatitis (ACD)

Groups of 8 female NMRI mice are sensitized on the shaved abdomen with 50 µl of oxazolone (2% in acetone) and challenged with 10 µl of 0.2% oxazolone on the inner surface of the right ear 7 days later. The unchallenged left ears serve as normal controls and dermatitis is evaluated from the individual differences in auricular weights, which are taken as a measure of inflammatory swelling 24 hours after the challenge. The test groups are treated with the test compounds orally (2 hours after challenge), the controls are treated similarly with the vehicles alone. For oral administration the compounds are administered in an oil in H₂O emulsion. Dermatitis is evaluated in test- and control groups. The animals are killed and both ears are cut off and weighed. Inhibitory activity of the test compounds is calculated from the differences in right and left ears (internal controls) in mice treated with test compounds compared with animals treated with the vehicle only. The data of the test- and the vehicle-treated control groups are statistically analyzed by ANOVA followed by Dunnet T-test (normal distribution or data) or by H and U-test, respectively. The compounds of the present invention inhibit the elicitation phase of allergic contact dermatitis based on the differences in auricular weights.

The compounds of the present invention are therefore indicated for use in the treatment or prevention of disorders, including diseases, mediated by interactions of LFA-1 with its ligands involved in cell adhesion, migration and activation.

Disorders, including diseases, mediated by interactions of LFA-1 with its ligands involved in cell adhesion, migration and activation include disorders associated with immune, inflammatory and allergic conditions, such as
- **disorders associated with inflammation**
   e.g. including (chronic) inflammatory disorders, disorders related with the inflammation of the bronchi, e.g. including bronchitis, cervix, e.g. including cervicitis, conjunctiva, e.g. conjunctivitis, esophagus, e.g. esophagitis, heart muscle, e.g. myocarditis, rectum, e.g. proctitis, sclera, e.g. scleritis, gums, involving bone, pulmonary inflammation (alveolitis), airways, e.g. asthma, such as bronchial asthma, acute respiratory distress syndrome (ARDS), inflammatory skin disorders such as contact hypersensitivity, (allergic) contact dermatitis, atopic dermatitis; fibrotic disease (e.g., pulmonary fibrosis), encephalitis, inflammatory osteolysis,
- **disorders associated with conditions of the immune system,**
   immune, such as autoimmune disorders e.g. including Graves' disease, Hashimoto's disease (chronic thyroiditis), multiple sclerosis, rheumatoid arthritis, arthritis, gout, osteoarthritis, scleroderma, lupus syndromes, systemic lupus erytomatosis, Sjoegren's syndrome, psoriasis, inflammatory bowel disease, including Crohn's disease, colitis, e.g. ulcerative colitis; sepsis, septic shock, autoimmune hemolytic anemia (AHA), autoantibody triggered urticaria, pemphigus, nephritis, glomerulonephritis, Goodpastur syndrom, ankylosing spondylitis, Reiter's syndrome, polymyositis, dermatomyositis, cytokine-mediated toxicity, interleukin-2 toxicity, alopecia, e.g. alopecia areata, hair growth, uveitis, lichen planus, bullous pemphigoid, myasthenia gravis, type I diabetes mellitus,immune-mediated infertility such as premature ovarian failure, polyglandular failure, hypothyroidism, pemphigus vulgaris, pemphigus I-oliaceus, paraneoplastic pemphigus, autoimmune hepatitis including that associated with hepatitis B virus (HBV) and hepatitis C virus (HCV), Addison's disease, autoimmune skin diseases, such as psoriasis, dermatitis herpetiformis, epidermolysis bullosa, linear IgA bullous dermatosis, epidermolysis bullosa acquisita, chronic bullous disease of childhood, pernicious anemia, hemolytic anemia, vitiligo, type I, type II and type III autoimmune polyglandular syndromes, Autoimmune Hypoparathyroidism, Autoimmune Hypophysitis, Autoimmune Oophoritis, Autoimmune Orchitis, pemphigoid gestationis, cicatricial pemphigoid, mixed essential cryoglobulinemia, immune thrombocytopenic purpura, Goodpasture's syndrome, autoimmune neutropenia, Eaton-Lambert myasthenic syndrome, stiff-man syndrome, encephalomyelitis, acute disseminated encephalomyelitis, Guillain-Barre syndrome, cerebellar degeneration, retinopathy, primary biliary sclerosis, sclerosing cholangitis autoimmune hepatitis, gluten-sensitive enteropathy, reactive arthritides, polymyositis/dermatomyositis, mixed connective tissue disease, Bechet's syndrome, polyarteritis nodosa allergic anguitis and granulomatosis (Churg-Strauss disease), polyangiitis overlap syndrome (hypersensitivity) vasculitis, Wegener's granulomatosis, temporal arteritis Kawasaki's disease, sarcoidosis, cryopathies, Celiac disease,
- **disorders associated with skin and connective tissue conditions**
   e.g. including eczema, atopic dermatitis, (allergic) contact dermatitis, psoriasis, acne, dermatomyositis, Sjörgen's syndrome, Churg-Struass syndrome, sunburn, skin cancer, urticaria, toxic epidermal necrolysis, age related skin conditions, cellulite,
- **disorders associated with allergic conditions,**
   e.g. including delayed-type hypersensitivity, allergic conjunctivitis, drug allergies, rhinitis, allergic rhinitis, vasculitis, contact dermatits;
- **disorders associated with infectious disorders, e.g. with chronic infectious conditions,**
   e.g. including bacterial disorders, otitis media, Lyme disease, thryoditis, viral disorders, parasitic disorders, fungal disorders, malaria, e.g. malaria anemia, sepsis, severe sepsis, septic shock, e.g. endotoxin-induced septic shock, exotoxin-induced toxic shock, infective (true septic) shock, septic shock caused by Gram-negative bacteria, pelvic inflammatory disease, AIDS, enteritis, pneumonia; meningitis, encephalitis,
- **disorders associated with transplantation,**
   e.g. including transplant rejection crisis and other disorders following transplantation, such as organ or tissue (xeno)transplant rejection, e.g. for the treatment of recipients of e.g. heart, lung, combined heart-lung, liver, kidney, pancreatic, skin, corneal transplants, graft versus host disease, such as following bone marrow transplantation, ischemic reperfusion injury.

The compound of the present invention are preferably useful for treatment of disorders associated with conditions of the immune system, inflammation and transplantation; e.g. including psoriasis, rheumatoid arthritis, inflammatory bowel diseases (Crohn's disease, ulcerative colitis), (systemic) lupus erythematosus, multiple sclerosis, Sjoegren's syndrom, rejection after transplantation and graft vs. host disease and inflammatory skin diseases, e.g. dermatitis, such as atopic dermatitis, e.g. allergic contact dermatitis.

In one embodiment the compounds of the present invention are useful in the treatment of autoimmune diseases, e.g. rheumatoid arthritis, psoriasis, inflammatory bowel disease, (systemic) lupus erythematosus, multiple sclerosis or of inflammatory (skin) diseases, e.g. dermatitis;
more preferably in the treatment of inflammatory bowel disease or rheumatoid arthritis; e.g. or dermatitis.

In another aspect the present invention provides
- a compound as defined in claim 5 or in claim 6 for use as a pharmaceutical for the treatment of disorders mediated by interactions of LFA-1 with its ligands involved in cell adhesion, migration and activation as defined in claim 5 or claim 6..

The disclosure describes also the use of a compound of the present invention as a pharmaceutical, e.g. for the treatment of disorders mediated by interactions of LFA-1 with its ligands involved in cell adhesion, migration and activation.

For pharmaceutical use one or more compounds of the present invention may be used, e.g. one, or a combination of two or more compounds of the present invention, preferably one compound of the present invention is used.

A compound of the present invention may be used as a pharmaceutical in the form of a pharmaceutical composition.

In another aspect the present disclosure describes a pharmaceutical composition comprising a compound of the present invention in association with at least one pharmaceutically acceptable excipient, e.g. appropriate carrier and/or diluent, e.g. including fillers, binders, disintegrants, flow conditioners, lubricants, sugars or sweeteners, fragrances, preservatives, stabilizers, wetting agents and/or emulsifiers, solubilizers, salts for regulating osmotic pressure and/or buffers.

In another aspect the present disclosure describes
- a pharmaceutical composition of the present invention for use of treating disorders which are mediated by interactions of LFA-1 with its ligands involved in cell adhesion, migration and activation;
- the use of a pharmaceutical composition of the present invention for treating disorders which are mediated by interactions of LFA-1 with its ligands involved in cell adhesion, migration and activation.

In a further aspect the present disclosure describes a method of treating disorders which are mediated by interactions of LFA-1 with its ligands involved in cell adhesion, migration and activation, which treatment comprises administering to a subject in need of such treatment a therapeutically effective amount of a compound of the present invention; e.g. in the form of a pharmaceutical composition.

In another aspect the present disclosure describes
a compound of the present invention for the manufacture of a medicament..

The invention, in another aspect, relates to the use of a compound as defined in claim 7 for the manufacture of a medicament,
e.g. a pharmaceutical composition,
for the treatment of disorders, which are mediated by interactions of LFA-1 with its ligands involved in cell adhesion, migration and activation as defined in claim 5.

Treatment of disorders (diseases) as used herein includes treatment and prophylaxis (prevention).

For such treatment, the appropriate dosage will, of course, vary depending upon, for example, the chemical nature and the pharmacokinetic data of a compound of the present invention used, the individual host, the mode of administration and the nature and severity of the conditions being treated. However, in general, for satisfactory results in larger mammals, for example humans, an indicated daily dosage includes a range
- from about 0.0001 g to about 1.5 g, such as 0.001 g to 1.5 g;
- from about 0.001 mg/kg body weight to about 20 mg/kg body weight, such as 0.01 mg/kg body weight to 20 mg/kg body weight,
for example administered in divided doses up to four times a day.

A compound of the present invention may be administered to larger mammals, for example humans, by similar modes of administration than conventionally used with other mediators, e.g. low molecular weight inhibitors, of activity of LFA-1 with its ligands involved in cell adhesion, migration and activation.

A compound of the present invention may be administered by any route, e.g. by any conventional route, for example enterally, e.g. including nasal, buccal, rectal, oral, administration; parenterally, e.g. including intravenous, intraarterial, intramuscular, intracardiac, subcutanous, intraosseous infusion, transdermal (diffusion through the intact skin), transmucosal (diffusion through a mucous membrane), inhalational administration; topically; e.g. including epicutaneous, intranasal, intratracheal administration; intraperitoneal (infusion or injection into the peritoneal cavity); epidural (peridural) (injection or infusion into the epidural space); intrathecal (injection or infusion into the cerebrospinal fluid); intravitreal (administration via the eye); or via medical devices, e.g. for local delivery, e.g. stents, e.g. in form of coated or uncoated tablets, capsules, (injectable) solutions, infusion solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.

For topical use, e.g. including administration to the eye, satisfactory results may be obtained with local administration of a 0.5-10 %, such as 1-3% concentration of active substance several times daily, e.g. 2 to 5 times daily.

The compounds of the present invention may be administered in the form of a pharmaceutically acceptable salt, or in free form; optionally in the form of a solvate. A compound of the present invention in the form of a salt and/or in the form of a solvate exhibit the same order of activity as a compound of the present invention in free form.

A compound of the present invention may be used for any method or use as described herein alone or in combination with one or more, at least one, other, second drug substance.

In another aspect the present disclosure describes
- A combination of a compound of the present invention with at least one second drug substance;
- A pharmaceutical combination comprising a compound of the present invention in combination with at least one second drug substance;
- A pharmaceutical composition comprising a compound of the present invention in combination with at least one second drug substance and one or more pharmaceutically acceptable excipient(s).;
- A compound of the present invention in combination with at least one second drug substance, e.g. in the form of a pharmaceutical combination or composition, for use in any method as defined herein, e.g.
- A combination, a pharmaceutical combination or a pharmaceutical composition, comprising a compound of the present invention and at least one second drug substance for use as a pharmaceutical;
- The use as a pharmaceutical of a compound of the present invention in combination with at least one second drug substance, e.g. in the form of a pharmaceutical combination or composistion;
- The use of a compound of the present invention for the manufacture of a medicament for use in combination with a second drug substance;
- A method for treating of disorders, which are mediated by interactions of LFA-1 with its ligands involved in cell adhesion, migration and activation, in a subject in need thereof, comprising co-administering, concomitantly or in sequence, a therapeutically effective amount of a compound of the present invention and at least one second drug substance, e.g. in the form of a pharmaceutical combination or composition;
- A compound of the present invention in combination with at least one second drug substance, e.g. in the form of a pharmaceutical combination or composition, for use in the preparation of a medicament for the treatment of disorders which are mediated by interactions of LFA-1 with its ligands involved in cell adhesion, migration and activation.

Combinations include fixed combinations, in which a compound of the present invention and at least one second drug substance are in the same formulation; kits, in which a compound of the present invention and at least one second drug substance in separate formulations are provided in the same package, e.g. with instruction for co-administration; and free combinations in which a compound of the present invention and at least one second drug substance are packaged separately, but instruction for concomitant or sequential administration are given.

In another aspect the present disclosure describes
- A pharmaceutical package comprising a first drug substance which is a compound of the present invention and at least one second drug substance, beside instructions for combined administration;
- A pharmaceutical package comprising a compound of the present invention beside instructions for combined administration with at least one second drug substance;
- A pharmaceutical package comprising at least one second drug substance beside instructions for combined administration with a compound of the present invention.

Treatment with may provide improvements compared with single treatment.

In another aspect the present disclosure describes
- A pharmaceutical combination comprising an amount of a compound of the present invention and an amount of a second drug substance, wherein the amounts are appropriate to produce a synergistic therapeutic effect;
- A method for improving the therapeutic utility of a compound of the present invention comprising co-administering, e.g. concomitantly or in sequence, of a therapeutically effective amount of a compound of the present invention and a second drug substance.
- A method for improving the therapeutic utility of a second drug substance comprising co-administering, e.g. concomitantly or in sequence, of a therapeutically effective amount of a compound of the present invention and a second drug substance.

A combination the present disclosure describes with a second drug substance as a combination partner may be administered by any conventional route, for example as set out above for a compound of the present invention. A second drug may be administered in dosages as appropriate, e.g. in dosage ranges which are similar to those used for single treatment, or, e.g. in case of synergy, even below conventional dosage ranges.

Pharmaceutical compositions the present disclosure describes may be manufactured according, e.g. analogously, to a method as conventional, e.g. by mixing, granulating, coating, dissolving or lyophilizing processes. Unit dosage forms may contain, for example, from about 0.1 mg to about 1500 mg, such as 1 mg to about 1000 mg.

Pharmaceutical compositions comprising a combination the present disclosure describes and pharmaceutical compositions comprising a second drug as described herein, may be provided as appropriate, e.g. according, e.g. analogously, to a method as conventional, or as described herein for a pharmaceutical composition the present disclosure describes.

By the term "second drug substance" is meant a chemotherapeutic drug, especially any chemotherapeutic agent other than a compound of the present invention, such as a compound of formula I.

For example, a second drug substance as used herein includes e.g.
- other compounds, than compounds of the present inventions which interact in cell adhesion, migration and activation activity of LFA-1 with its ligands, e.g. including antibodies and low molecular weight compounds,
- anti-inflammatory and/or immunomodulatory drugs,
- anti-allergic drugs.

Anti-inflammatory and/or immunomodulatory drugs which are prone to be useful in combination with a compound of the present invention include e.g.
- mediators, e.g. inhibitors, of mTOR activity, including rapamycin of formula 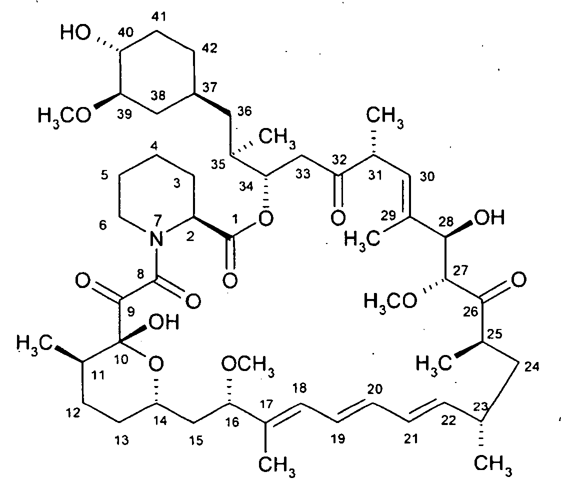 and rapamycin derivatives, e.g. including
   40-O-alkyl-rapamycin derivatives, such as 40-O-hydroxyalkyl-rapamycin derivatives, such as 40-O-(2-hydroxy)-ethyl-rapamycin (everolimus),
   32-deoxo-rapamycin derivatives and 32-hydroxy-rapamycin derivatives, such as 32-deoxorapamycin,
   16-O-substituted rapamycin derivatives such as 16-pent-2-ynyloxy-32-deoxorapamycin,
   16-pent-2-ynyloxy-32 (S or R) -dihydro-rapamycin, 16-pent-2-ynyloxy-32(S or R)-dihydro-40-O-(2-hydroxyethyl)-rapamycin, rapamycin derivatives which are acylated at the oxygen group in position 40, e.g. 40-[3-hydroxy-2-(hydroxy-methyl)-2-methylpropanoate]-rapamycin (also known as CCl779), rapamycin derivatives which are substituted in 40 position by heterocyclyl, e.g. 40-epi-(tetrazolyl)-rapamycin (also known as ABT578),
   the so-called rapalogs, e. g. as disclosed in WO9802441, WO0114387 and WO0364383, such as AP23573, and
   compounds disclosed under the name TAFA-93 AP23464, AP23675, AP23841 and biolimus (e.g. biolimus A9).
- mediators, e.g. inhibitors, of calcineurin, e.g. cyclosporin A, FK 506;
- ascomycins having immuno-suppressive properties, e.g. ABT-281, ASM981;
- corticosteroids; cyclophosphamide; azathioprene; leflunomide; mizoribine;
- mycophenolic acid or salt; mycophenolate mofetil;
- 15-deoxyspergualine or an immunosuppressive homologue, analogue or derivative thereof;
- mediators, e.g. inhibitors, of bcr-abl tyrosine kinase activity;
- mediators, e.g. inhibitors, of c-kit receptor tyrosine kinase activity;
- mediators, e.g. inhibitors, of PDGF receptor tyrosine kinase activity, e.g. Gleevec (imatinib);
- mediators, e.g. inhibitors, of p38 MAP kinase activity,
- mediators, e.g. inhibitors, of VEGF receptor tyrosine kinase activity,
- mediators, e.g. inhibitors, of PKC activity, e.g. as disclosed in WO0238561 or WO0382859, e.g. the compound of Example 56 or 70;
- mediators, e.g. inhibitors, of JAK3 kinase activity, e.g. N-benzyl-3,4-dihydroxy-benzylidene-cyanoacetamide α-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550), or a compound as disclosed in WO2004052359 or WO2005066156;
- mediators, e.g. agonists or modulators of S1P receptor activity, e.g. FTY720 optionally phosphorylated or an analog thereof, e.g. 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-benzyl}-azetidine-3-carboxylic acid or its pharmaceutically acceptable salts;
- immunosuppressive monoclonal antibodies, e.g., monoclonal antibodies to leukocyte receptors, e.g., Blys/BAFF receptor, MHC, CD2, CD3, CD4, CD7, CD8, CD25, CD28, CD40, CD45, CD52, CD58, CD80, CD86, IL-12 receptor, IL-17 receptor, IL-23 receptor or their ligands;
- other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a mutant thereof, e.g. LEA29Y;
- mediators, e.g. inhibitors of adhesion molecule activities, e.g. LFA-1 antagonists, ICAM-1 or -3 antagonists, VCAM-4 antagonists or VLA-4 antagonists,
- mediators, e.g. antagonists of CCR9 acitiviy,
- mediators, e.g. inhibitors, of MIF activity,
- 5-aminosalicylate (5-ASA) agents, such as sulfasalazine, Azulfidine®, Asacol®, Dipentum®, Pentasa®, Rowasa®, Canasa®, Colazal®, e.g. drugs containing mesalamine; e.g mesalazine in combination with heparin;
- mediators, e.g. inhibitors, of TNF-alpha activity, e.g. including antibodies which bind to TNF-alpha, e.g. infliximab (Remicade®), thalidomide, lenalidomide,
- nitric oxide releasing non-steriodal anti-inlammatory drugs (NSAIDs), e.g. including COX-inhibiting NO-donating drugs (CINOD);
- phospordiesterase, e.g. mediatorssuch as inhibitors of PDE4B activity,
- mediators, e.g. inhibitors, of caspase activity,
- mediators, e.g. agonists, of the G protein coupled receptor GPBAR1,
- mediators, e.g. inhibitors, of ceramide kinase activity,
- 'multi-functional anti-inflammatory' drugs (MFAIDs), e.g. cytosolic phoshpholipase A2 (cPLA2) inhibitors, such as membrane-anchored phospholipase A2 inhibitors linked to glycosaminoglycans;
- antibiotics, such as penicillins, cephalosporins, erythromycins, tetracyclines, sulfonamides, such as sulfadiazine, sulfisoxazole; sulfones, such as dapsone; pleuromutilins, fluoroquinolones, e.g. metronidazole, quinolones such as ciprofloxacin; levofloxacin; probiotics and commensal bacteria e.g. Lactobacillus, Lactobacillus reuteri;
- antiviral drugs, such as ribivirin, vidarabine, acyclovir, ganciclovir, zanamivir, oseltamivir phosphate, famciclovir, atazanavir, amantadine, didanosine, efavirenz, foscarnet, indinavir, lamivudine, nelfinavir, ritonavir, saquinavir, stavudine, valacyclovir, valganciclovir, zidovudine.

Anti-inflammatory which are prone to be useful in combination with a compound of the present invention include e.g. non-steroidal antiinflammatory agents (NSAIDs) such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone); cyclooxygenase-2 (COX- 2) inhibitors such as celecoxib; inhibitors of phosphodiesterase type IV (PDE-IV); antagonists of the chemokine receptors, especially CCR-1, CCR-2, and CCR-3; cholesterol lowering agents such as HMG-CoA reductase inhibitors (lovastatin, simvastatin and pravastatin, fluvastatin, atorvastatin, and other statins), sequestrants (cholestyramine and colestipol), nicotinic acid, fenofibric acid derivatives (gemfibrozil, clofibrat, fenofibrate and benzafibrate), and probucol; anticholinergic agents such as muscarinic antagonists (ipratropium bromide); other compounds such as theophylline, sulfasalazine and aminosalicylates, e.g. 5-aminosalicylic acid and prodrugs thereof, antirheumatics.

Antiallergic drugs which are prone to be useful in combination with a compound of the present invention include e.g. antihistamines (H1-histamine antagonists), e.g. bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, fexofenadine, descarboethoxyloratadine, and non-steroidal anti- asthmatics such as β2-agonists (terbutaline, metaproterenol, fenoterol, isoetharine, albuterol, bitolterol, salmeterol and pirbuterol), theophylline, cromolyn sodium, atropine, ipratropium bromide, leukotriene antagonists (zafirlukast, montelukast, pranlukast, iralukast, pobilukast, SKB-106,203), leukotriene biosynthesis inhibitors (zileuton, BAY-1005); bronchodilators, antiasthmatics (mast cell stabilizers).

The weight ratio of a compound of the present invention to other drug may vary, e.g. dependent on the activity of the combination partners used, the kind of disease treated, and will further depend upon the effective dose of each ingredient and may be established e.g. by instructions given for the other drug and testing, e.g. according, e.g. analogously, to a method as conventional.

The chemical names of the compounds of the present invention as indicated herein are copied from ISIS, version 2.5 (AutoNom 2000 Name).

In the following Examples all temperatures indicated are in degree Celsius (°C).

The following abbreviations are used:
- EtOAc: ethyl acetate
- rt: room temperature
- THF: tetrahydrofurane

### Example 1

### (3aS,5R)-3a-(4-Bromo-benzyl)-5-(tert-butyl-dimethyl-silanyloxy)-2-(3,5-dichlorophenyl)-1,1-dioxo-3a,4,5,6-tetrahydro-1H-1lambda*6*-pyrrolo[1,2-b]isothiazol-3-ol

### A) (2S,R)-4-(tert-Butyl-dimethyl-silanyloxy)-pyrrolidine-1,2-dicarboxylic acid 1-allyl ester 2-methyl ester

To a solution of 20 g of (2S,4S)-4-azido-pyrrolidine-1,2-dicarboxylic acid 1-allyl ester 2-methyl ester and 14.85 g imidazole in 300 ml of CH₃CN are added 26.3 g of (tert.butyl)(dimethyl)silylchloride in one portion and the mixture obtained is stirred for 1 hour at rt. To the mixture obtained EtOAc and half saturated aqeous NaCl solution are added, the phase obtained are separated, the organic layer obtained is washed, dried and from, the mixture obtained solvent is evaporated. The evaopration residue obtained is subjected to chromatography (2S,4R)-4-(tert-Butyl-dimethyl-silanyloxy)-pyrrolidine-1,2-dicarboxylic acid 1-allyl ester 2-methyl ester is obtained in the form of a colorless oil.
MS: 366/709 (MNa⁺/2MNa⁺), ¹H-NMR and ¹³C-NMR data are in accordance with the proposed structure.

### Ba) (2S,4R)-2-(4-Bromo-benzyl)-4-(tert-butyl-dimethyl-silanyloxy)-pyrrolidine-1,2-dicarboxylic acid 1-allyl ester 2-methyl ester, and

### Bb) (2R,4R)-2-(4-Bromo-benzyl)-4-(tert-butyl-dimethyl-silanyloxy)-pyrrolidine-1,2-dicarboxylic acid 1-allyl ester 2-methyl ester

Compounds of formula

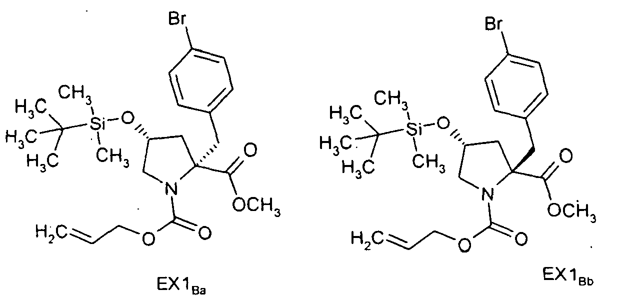

To a solution of 10 g of (2S,4R)-4-(tert.butyl)(dimethyl)silanytoxy)-pyrrolidine-1,2-dicarboxylic acid 1-allyl ester 2-methyl ester and 14.55 g of 4-bromobenzylbromide in 100 ml of THF are added 11.61 g of solid potassium hexamethyl disilazane in such a rate that the temperature is kept below 40°. The mixture obtained is stirred at rt for 20 minutes and 1 N aqueous HCl and EtOAc are added. The phases obtained are separated and the organic layer obtained is washed, dried and solvent is evaporated. The evaporation resisue obtained is subjected to chromatography. (2S,4R)-2-(4-Bromo-benzyl)-4-(tert-butyl-dimethyl-silanyloxy)-pyrrolidine-1,2-dicarboxylic acid 1-allyl ester 2-methyl ester, (3.3 g) and (2R,4R)-2-(4-Bromo-benzyl)-4-(tert-butyl-dimethyl-silanyloxy)-pyrrolidine-1,2-dicarboxylic acid 1-allyl ester 2-methyl ester (9.1 g) are obtained. Both compounds: MS: 535/1047 (MNa⁺/2MNa⁺); ¹H-NMR and ¹³C-NMR data are in accordance with the proposed structure.

### C) (2S,4R)-2-(4-Bromo-benzyl)-4-(tert-butyl-dimethyl-silanyloxy)-pyrrolidine-2-carboxylic acid methyl ester

A compound of formula

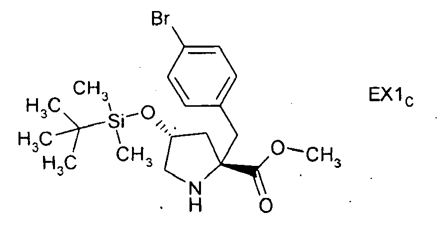

A mixture of 2.81 g of compound (2S,4R)-2-(4-bromo-benzyl)-4-(tert-butyl-dimethyl-silanyloxy)-pyrrolidine-1,2-dicarboxylic acid 1-allyl ester 2-methyl ester, 3.24 g of 1,4-diaza-bicyclo[2.2.2]octane and 653 mg of tetrakis-(triphenylphosphine)-palladium in 30 ml of CH₂Cl₂ is stirred at rt for 25 minutes. EtOAc and an aqueous, saturated NaHCO₃ solution are added, The phases obtained are separated, the organic layer obtained is dried and solvent is evaporated The evaporation residue obtained is subjected to chromatograph.
(2S,4R)-2-(4-Bromo-benzyl)-4-(tert-butyl-dimethyl-silanyloxy)-pyrrolidine-2-carboxylic acid methyl ester is obtained in the form of an oil.
MS:: 451 (MNa⁺), ¹H-NMR and ¹³C-NMR data are in accordance with the proposed structure

### D) (2S,4R)-2-(4-Bromo-benzyl)-4-(tert-butyl-dimethyl-silanyloxy)-1-(3,5-dichlorophenylmethanesulfonyl)-pyrrolidine-2-carboxylic acid methyl ester

A compound of formula

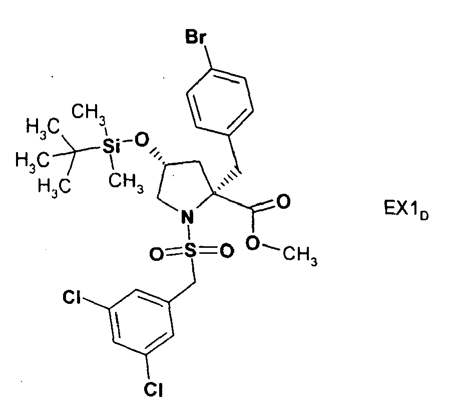

To a solution of 400 mg of (2S,4R)-2-(4-bromo-benzyl)-4-(tert-butyl-dimethyl-silanyloxy)-pyrrolidine-2-carboxylic acid methyl ester and 1.5 g of 4-dimethylamino pyridine (DMAP, Steglich base) in 20 ml of CH₃CN are added 600 mg of 2,5-dichlorophenyl methanesulfonyl chloride at rt and the mixture obtained is stirred at rt. To the mixture obtained EtOAc and a saturated aqueous NAHCO₃-solution is added, the phases obtained are separated and the organic layer is washed, dried, and solvent is evaporated. The evaporation residue obtained is subjected to chromatography. (2S,4R)-2-(4-Bromo-benzyl)-4-(tert-butyl-dimethyl-silanyloxy)-1-(3,5-dichloro-phenylmethanesulfonyl)-pyrrolidine-2-carboxylic acid methyl ester is obtained in the fornm of a slightly yellow oil.
MS:: 673 (MNa⁺), ¹H-NMR and ¹³C-NMR data are in accordance with the proposed structure.

### E) (3aS,5R)-3a-(4-Bromo-benzyl)-5-(tert-butyl-dimethyl-silanyloxy)-2-(3 5-dichloro-phenyl)

### 1,1-dioxo-3a,4,5,6-tetrahydro-1H-1lambda*6*-pyrrolo[1,2-b]isothiazol-3-ol

A compound of formula

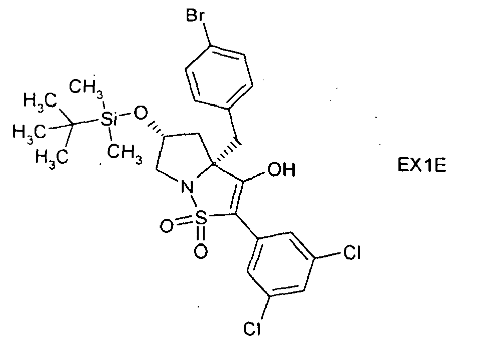

To a solution of 460 mg of (2S,4R)-2-(4-bromo-benzyl)-4-(tert-butyl-dimethyl-silanyloxy)-1-(3,5-dichloro-phenylmethanesulfonyl)-pyrrolidine-2-carboxylic acid methyl ester in 15 ml of THF are added 222 mg of solid potassium hexamethyl disilazane in one portion at rt. The mixture obtained is stirred for 15 minutes at rt and EtOAc and 1 N HCl are added. The phases obtained are separated, the organic layer obtained is washed, dried and solvent is evaporated. (3aS,5R)-3a-(4-Bromo-benzyl)-5-(tert-butyl-dimethyl-silanyloxy)-2-(3,5-dichlorophenyl)-1,1-dioxo-3a,4,5,6-tetrahydro-1H-1lambda*6*-pyrrolo[1,2-b]isothiazol-3-ol is obtained in the form of a crystalline solid.
MS, ¹H-NMR and ¹³C-NMR data are in accordance with the proposed structure.

### Example 2

### (3aS,5R)-3a-(4-Bromo-benzyl)-5-(tert-butyl-dimethyl-silanyloxy)-2-(3,5-dichlorophenyl)-3-methoxy-3a,4,5,6-tetrahydro-pyrrolo[1,2-b]isothiazole 1,1-dioxide

A compound of formula

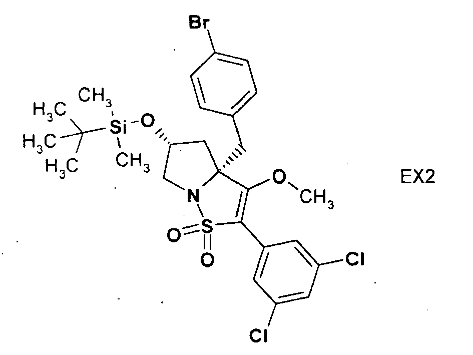

An approx 1 M solution of diazomethane in diethylether is added to a solution of 410 mg of (3aS,5R)-3a-(4-bromo-benzyl)-5-(tert-butyl-dimethyl-silanyloxy)-2-(3,5-dichloro-phenyl)-1,1-dioxo-3a,4,5,6-tetrahydro-1H-1lambda*6*-pyrrolo[1,2-b]isothiazol-3-ol in 20 ml of EtOAc until the characteristic yellow color of diazomethane does not longer disappear. From the mixture obtained solvent is evaporated and the evaporation residue is subceted to chromatography (3aS,5R)-3a-(4-Bromo-benzyl)-5-(tert-butyl-dimethyl-silanyloxy)-2-(3,5-dichloro-phenyl)-3-methoxy-3a,4,5,6-tetrahydro-pyrrolo[1,2-b]isothiazole 1,1-dioxide is obtained in the form of an amorphous powder.
MS:: 654 (MNa⁺), ¹H-NMR and ¹³C-NMR data are in accordance with the proposed structure

### Example 3

### (3aS,5R)-3a-(4-Bromo-benzyl)-2-(3,5-dichloro-phenyl)-3-methoxy-1,1-dioxo-3a,4,5,6-tetrahydro-1H-1lambda*6*-pyrrolo[1,2-b]isothiazol-5-ol

A compound of formula

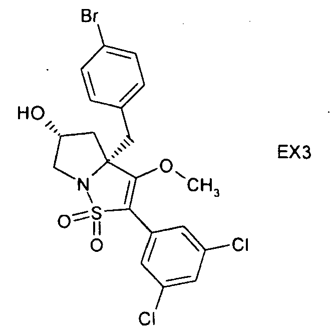

To a solution of 280 mg of (3aS,5R)-3a-(4-bromo-benzyl)-5-(tert-butyl-dimethyl-silanyloxy)-2-(3,5-dichloro-phenyl)-3-methoxy-3a,4,5,6-tetrahydro-pyrrolo[1,2-b]isothiazole 1,1-dioxide in 8 ml of CH₃CN are added 3 ml of aqueous (40 v/v%) hydrogen fluoride solution and the mixture obtained is stirred for 70 minutes at rt. To the mixture obtained EtOAc and an aqueous solution of saturated NaHCO₃ are added and the phases obtained are separated. The organic layer obtained is washed, dried and solvent is evaporated. The evaporation residue obtained is subjected to chromatography. (3aS,5R)-3a-(4-Bromo-benzyl)-2-(3,5-dichloro-phenyl)-3-methoxy-1,1-dioxo-3a,4,5,6-tetrahydro-1H-1lambda*6*-pyrrolo[1,2-b]isothiazol-5-ol is obtained in the form of an amorphous powder.
MS: 530/1055 (MNa⁺/2MNa⁺), ¹H-NMR and ¹³C-NMR data of any compound described are in accordance with the proposed structure:

### Example 4

### (S)-3a-(4-Bromo-benzyl)-2-(3,5-dichloro-phenyl)-3-methoxy-1,1-dioxo-3a,4-dihydro-1H-1lambda*6*-pyrrolo[1,2-b]isothiazol-5-one

A compound of formula

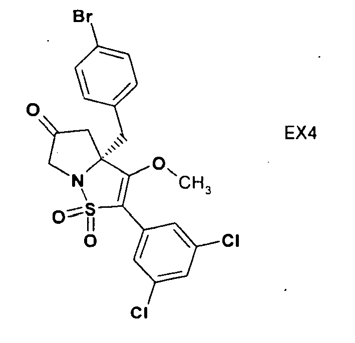

A mixture of 83 mg of 1-hydroxy-1,2-benziodoxol-3(1H)-one 1-oxide (IBX, CAS Regno: 444722-55-8) and 50 mg of (3aS,5R)-3a-(4-bromo-benzyl)-2-(3,5-dichloro-phenyl)-3-methoxy-1,1-dioxo-3a,4,5,6-tetrahydro-1H-1lambda*6*-pyrrolo[1,2-b]isothiazol-5-ol in 5 ml EtOAc are reacted under microwave conditions (100°; 65 minutes; Personal chemistry Emrys optimizer). The mixture obtained is filtrated (removal of excess reagent), the filtrate obtained is concentrated at reduced pressure and the concentration residue obtained is subjected to chromatography. (S)-3a-(4-Bromo-benzyl)-2-(3,5-dichloro-phenyl)-3-methoxy-1,1-dioxo-3a,4-dihydro-1H-1lambda*6*-pyrrolo[1,2-b]isothiazol-5-one is obtained in the form of an amorphous solid.
MS: 537 (MNa⁺), ¹H-NMR and ¹³C-NMR data of any compound described are in accordance with the proposed structure.

### Example 5

According to a method as set out in any of the previous Examples the compound of formula 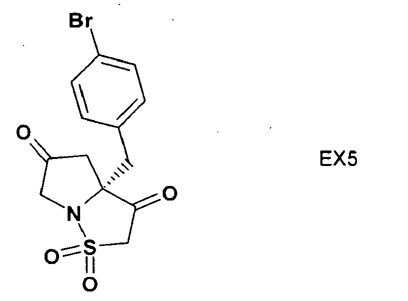 is obtained. MS, ¹H-NMR and ¹³C-NMR data are in accordance with the proposed structure.

## Claims

1. A compound of formula 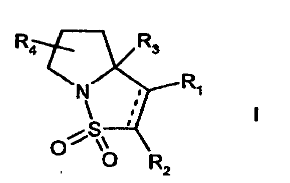 wherein
the dotted line is a bond or is no bond,
R₁ is hydrogen, optionally substituted alkyl, alkenyl, alkynyl, cycloalkyl, alkoxy, aryl or heterocyclyl, hydroxy, SH, SR₅, cyano, halogen or amino, or
the dotted line is no bond and R₁ is attached to the ring system via a double bond and is oxo,
R₂ is hydrogen or optionally substituted cycloalkyl, aryl, or heterocyclyl,
R₃ is hydrogen, COOR₆, aminocarbonyl, or optionally substituted alkyl, alkenyl, alkynyl, aralkyl, alkoxy, cycloalkyloxy, aryloxy, or heterocycyloxy,
R₄ is hydrogen, halogen, hydroxy, SH, optionally substituted alkyl, alkenyl, alkynyl, alkoxy or alkylthio, or a silyl group such as trialkylsilyl or trialkylsilyloxy, e.g. tri(C₁₋₈)-alkylsilyl(oxy), N₃, amino, or
R₄ is heterocyclyl comprising at least one nitrogen atom as a heteroatom and being bound via that nitrogen atom to a compound of formula I, or
R₄ is attached to the ring system by a double bond and is oxo; and
R₅ and R₆ independently of each other are alkyl, alkenyl, alkynyl, cycloalkyl, aryl or heterocyclyl,
with the exception of (6S)-6-(tert-butyl-diphenyl-silanyloxymethyl)-3a,4,5,6-tetrahydro-pyrrolo[1,2-b]isothiazole 1,1-dioxide, (6S)-(1,1-dioxo-3a,4,5,6-tetrahydro-1H-1λ⁶-pyrrolo[1,2-b]isothiazol-6-yl)-methanol and (3aS)-2,3,3a,4,5,6-hexahydropyrrolo[1,2-b]isothiazole 1,1-dioxide.

2. A compound according to claim 1, wherein a compound of formula I is a compound of formula

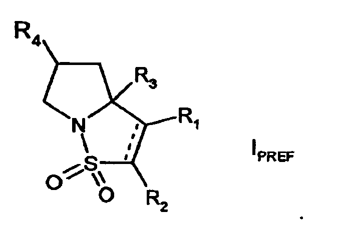

3. A compound according to any one of claims 1 or 2 wherein
R₁ is (C₁₋₈)alkoxy, or R₁ is attached to the ring system via a double bond and is oxo, R₂ is substituted phenyl,
R₃ is substituted phenylmethyl, and
R₄ is hydroxy, trialkylsilyloxy, or R₄ is attached via a double bond and is oxo.

4. A compound according to any one of claims 1 to 3 in the form of a salt.

5. A compound of formula 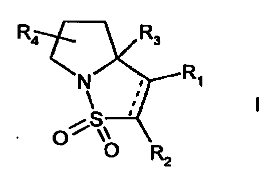 wherein
the dotted line is a bond or is no bond,
R₁ is hydrogen, optionally substituted alkyl, alkenyl, alkynyl, cycloalkyl, alkoxy, aryl or heterocyclyl, hydroxy, SH, SR₅, cyano, halogen or amino, or
the dotted line is no bond and R₁ is attached to the ring system via a double bond and is oxo,
R₂ is hydrogen or optionally substituted cycloalkyl, aryl, or heterocyclyl,
R₃ is hydrogen, COOR₆, aminocarbonyl, or optionally substituted alkyl, alkenyl, alkynyl, aralkyl, alkoxy, cycloalkyloxy, aryloxy, or heterocycyloxy,
R₄ is hydrogen, halogen, hydroxy, SH, optionally substituted alkyl, alkenyl, alkynyl, alkoxy or alkylthio, or a silyl group such as trialkylsilyl or trialkylsilyloxy, e.g. tri(C₁₋₆)alkylsilyl(oxy), N₃, amino, or
R₄ is heterocyclyl comprising at least one nitrogen atom as a heteroatom and being bound via that nitrogen atom to a compound of formula I, or
R₄ is attached to the ring system by a double bond and is oxo; and
R₅ and R₆ independently of each other are alkyl, alkenyl, alkynyl, cycloalkyl, aryl or heterocyclyl,
or a pharmaceutically acceptable salt thereof, for use in the treatment or prevention of a disorder mediated by LFA-1 with its ligands involved in cell adhesion, migration and activation, where said disorder is selected from immune, inflammatory and allergic conditions.

6. A compound of formula 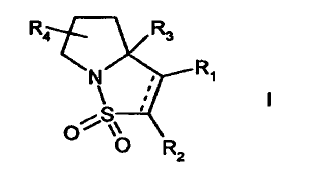 wherein
R₁ is (C₁₋₈)alkoxy, or R₁ is attached to the ring system via a double bond and is oxo, R₂ is substituted phenyl,
R₃ is substituted phenylmethyl, and
R₄ is hydroxy, trialkylsilyloxy, or R_{4EX} is attached via a double bond and is oxo, or a pharmaceutically acceptable salt thereof, for use according to claim 5.

7. The use of a compound of formula 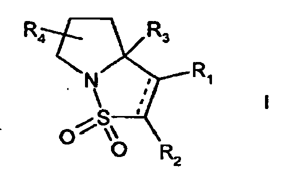 wherein
the dotted line is a bond or is no bond,
R₁ is hydrogen, optionally substituted alkyl, alkenyl, alkynyl, cycloalkyl, alkoxy, aryl or heterocyclyl, hydroxy, SH, SR₅, cyano, halogen or amino, or
the dotted line is no bond and R₁ is attached to the ring system via a double bond and is oxo,
R₂ is hydrogen or optionally substituted cycloalkyl, aryl, or heterocyclyl,
R₃ is hydrogen, COOR₆, aminocarbonyl, or optionally substituted alkyl, alkenyl, alkynyl, aralkyl, alkoxy, cycloalkyloxy, aryloxy, or heterocycyloxy,
R₄ is hydrogen, halogen, hydroxy, SH, optionally substituted alkyl, alkenyl, alkynyl, alkoxy or alkylthio, or a silyl group such as trialkylsilyl or trialkylsilyloxy, e.g. tri(C₁₋₆)alkylsilyl(oxy), N₃, amino, or
R₄ is heterocyclyl comprising at least one nitrogen atom as a heteroatom and being bound via that nitrogen atom to a compound of formula I, or
R₄ is attached to the ring system by a double bond and is oxo; and
R₅ and R₆ independently of each other are alkyl, alkenyl, alkynyl, cycloalkyl, aryl or heterocyclyl,
or a pharmaceutically acceptable salt thereof, for the manufacture of a medicament for the treatment of disorders mentioned in claim 5.

## Patentansprüche

1. Verbindung der Formel 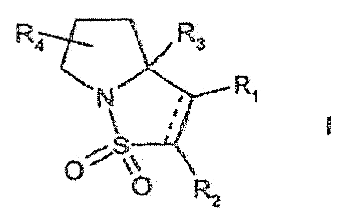 worin
die gestrichelte Linie für eine Bindung oder keine Bindung steht,
R₁ für Wasserstoff, gegebenenfalls substituiertes Alkyl, Alkenyl, Alkinyl, Cycloalkyl, Alkoxy, Aryl oder Heterocyclyl, Hydroxy, SH, SR₅, Cyano, Halogen oder Amino steht oder
die gestrichelte Linie für keine Bindung steht und R₁ über eine Doppelbindung an das Ringsystem gebunden ist und für Oxo steht,
R₂ für Wasserstoff oder gegebenenfalls substituiertes Cycloalkyl, Aryl oder Heterocyclyl steht,
R₃ für Wasserstoff, COOR₆, Aminocarbonyl oder gegebenenfalls substituiertes Alkyl, Alkenyl, Alkinyl, Aralkyl, Alkoxy, Cycloalkyloxy, Acyloxy oder Heterocyclyloxy steht,
R₄ für Wasserstoff, Halogen, Hydroxy, SH, gegebenenfalls substituiertes Alkyl, Alkenyl, Alkinyl, Alkoxy oder Alkylthio oder eine Silylgruppe wie Trialkylsilyl oder Trialkylsilyloxy, z.B. Tri(C₁-₆)-alkylsilyl(oxy), N₃ oder Amino steht oder
R₄ für Heterocyclyl steht, das mindestens ein Stickstoffatom als Heteroatom enthält und über dieses Stickstoffatom an eine Verbindung der Formel I gebunden ist, oder
R₄ über eine Doppelbindung an das Ringsystem gebunden ist und für Oxo steht und
R₅ und R₆ unabhangig voneinander für Alkyl, Alkenyl, Alkinyl, Cycloalkyl, Aryl oder Heterocyclyl stehen,
mit Ausnahme von (6S)-6-(tert.-Butyldiphenyl-silanyloxymethyl)-3a,4,5,6-tetrahydropyrrolo[1,2-b]isothiazol-1,1-dioxid, (6S)-(1,1-Dioxo-3a,4,5,6-tetrahydro-1H-1λ⁶-pyrrolo[1,2-b]isothiazol-6-yl)-methanol und (3aS)-2,3,3a,4,5,6-Hexahydropyrrolo-[1,2-b]isothiazol-1,1-dioxid.

2. Verbindung nach Anspruch 1, wobei es sich bei einer Verbindung der Formel I um eine Verbindung der Formel 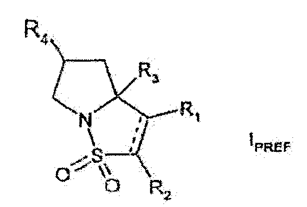 handelt.

3. Verbindung nach einem der Ansprüche 1 oder 2, worin
R₁ für (C₁₋₈)-Alkoxy steht oder R₁ über eine Doppelbindung an das Ringsystem gebunden ist und für Oxo steht,
R₂ für substituiertes Phenyl steht,
R₃ für substituiertes Phenylmethyl steht und R₄ für Hydroxy oder Trialkylsilyloxy steht oder R₄ über eine Doppelbindung an das Ringsystem gebunden ist und für Oxo steht.

4. Verbindung nach einem der Ansprüche 1 bis 3 in Form eines Salzes.

5. Verbindung der Formel 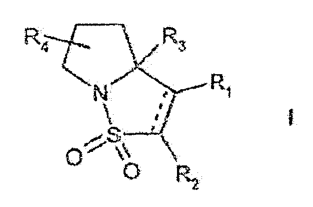 worin
die gestrichelte Linie für eine Bindung oder keine Bindung steht,
R₁ für Wasserstoff, gegebenenfalls substituiertes Alkyl, Alkenyl, Alkinyl, Cycloalkyl, Alkoxy, Aryl oder Heterocyclyl, Hydroxy, SH, SR₅, Cyano, Halogen oder Amino steht oder
die gestrichelte Linie für keine Bindung steht und R₁ über eine Doppelbindung an das Ringsystem gebunden ist und für Oxo steht,
R₂ für Wasserstoff oder gegebenenfalls substituiertes Cycloalkyl, Aryl oder Heterocyclyl steht,
R₃ für Wasserstoff, COOR₆, Aminocarbonyl oder gegebenenfalls substituiertes Alkyl, Alkenyl, Alkinyl, Aralkyl, Alkoxy, Cycloalkyloxy, Acyloxy oder Heterocyclyloxy steht,
R₄ für Wasserstoff, Halogen, Hydroxy, SH, gegebenenfalls substituiertes Alkyl, Alkenyl, Alkinyl, Alkoxy oder Alkylthio oder eine Silylgruppe wie Trialkylsilyl oder Trialkylsilyloxy, z.B. Tri-(C₁₋₆)-alkylsilyl(oxy), N₃ oder Amino steht oder
R₄ für Heterocyclyl steht, das mindestens ein Stickstoffatom als Heteroatom enthält und über dieses Stickstoffatom an eine Verbindung der Formel I gebunden ist, oder
R₄ über eine Doppelbindung an das Ringsystem gebunden ist und für Oxo steht und
R₅ und R₆ unabhängig voneinander für Alkyl, Alkenyl, Alkinyl, Cycloalkyl, Aryl oder Heterocyclyl stehen,
oder ein pharmazeutisch unbedenkliches Salz davon zur Verwendung bei der Behandlung oder Prävention einer durch LFA-1 mit seinen Liganden, die an Zelladhäsion, -migration und -aktivierung beteiligt sind, vermittelten Störung, wobei die Störung unter Immunleiden, entzündlichen Leiden und allergischen Leiden ausgewählt ist.

6. Verbindung der Formel 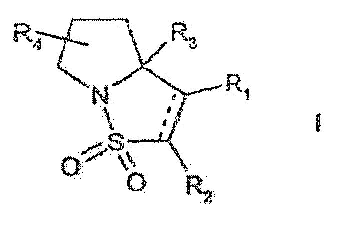 worin
R₁ für (C₁₋₈)-Alkoxy steht oder R₁ über eine Doppelbindung an das Ringsystem gebunden ist und für Oxo steht,
R₂ für substituiertes Phenyl steht,
R₃ für substituiertes Phenylmethyl steht und R₄ für Hydroxy oder Trialkylsilyloxy steht oder R_{4EX} über eine Doppelbindung gebunden ist und für Oxo steht,
oder ein pharmazeutisch unbedenkliches Salz davon zur Verwendung nach Anspruch 5.

7. Verwendung einer Verbindung der Formel 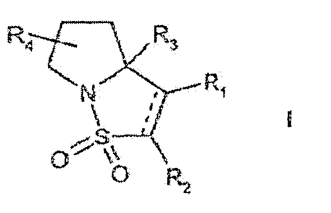 worin
die gestrichelte Linie für eine Bindung oder keine Bindung steht,
R₁ für Wasserstoff, gegebenenfalls substituiertes Alkyl, Alkenyl, Alkinyl, Cycloalkyl, Alkoxy, Aryl oder Heterocyclyl, Hydroxy, SH, SR₅, Cyano, Halogen oder Amino steht oder
die gestrichelte Linie für keine Bindung steht und R₁ über eine Doppelbindung an das Ringsystem gebunden ist und für Oxo steht,
R₂ für Wasserstoff oder gegebenenfalls substituiertes Cycloalkyl, Aryl oder Heterocyclyl steht,
R₃ für Wasserstoff, COOR₆, Aminocarbonyl oder gegebenenfalls substituiertes Alkyl, Alkenyl, Alkinyl, Aralkyl, Alkoxy, Cycloalkyloxy, Aryloxy oder Heterocyclyloxy steht,
R₄ für Wasserstoff, Halogen, Hydroxy, SH, gegebenenfalls substituiertes Alkyl, Alkenyl, Alkinol, Alkoxy oder Alkylthio oder eilne Silylgruppe wie Trialkylsilyl oder Trialkylsilyloxy, z.B. Tri(C₁₋₆)-alkylsilyl(oxy), N₃ oder Amino steht oder
R₄ für Heterocyclyl steht, das mindestens ein Stickstoffatom als Heteroatom enthält und über dieses Stickstoffatom an eine Verbindung der Formel I gebunden ist, oder
R₄ über eine Doppelbindung an das Ringsystem gebunden ist und für Oxo steht und
R₅ und R₅ unabhängig voneinander für Alkyl, Alkenyl, Alkinyl, Cycloalkyl, Aryl oder Heterocyclyl stehen,
oder eines pharmazeutisch unbedenklichen Salzes davon zur Herstellung eines Arzneimittels zur Behandlung von in Anspruch 5 aufgeführten Störungen.

## Revendications

1. Composé de formule 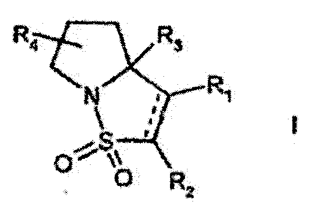 dans laquelle
la ligne en pointillé est une liaison ou n'est pas une liaison,
R₁ est hydrogène, alkyle, alcényle, alcynyle, cycloalkyle, alcoxy, aryle ou hétérocyclyle éventuellement substitué, hydroxy, SH, SR₅, cyano, halogène ou amino, ou
la ligne en pointillé n'est pas une liaison et R₁ est lié au système de cycle par l'intermédiaire d'une double liaison et est oxo,
R₂ est hydrogène ou cycloalkyle, aryle ou hétérocyclyle éventuellement substitué,
R₃ est hydrogène, COOR₆, aminocarbonyle, ou alkyle, alcényle, alcynyle, aralkyle, alcoxy, cycloalkyloxy, aryloxy ou hétérocyclyloxy éventuellement substitué,
R₄ est hydrogène, halogène, hydroxy, SH, alkyle, alcényle, alcynyle, alcoxy ou alkylthio éventuellement substitué, ou un groupement silyle tel que trialkylsilyle ou trialkylsilyloxy, par exemple tri(C₁₋₆)alkylsilyl(oxy), N₃, amino, ou
R₄ est hétérocyclyle comprenant au moins un atome d'azote en tant qu'hétéroatome et étant lié par l'intermédiaire de cet atome d'azote à un composé de formule I, ou
R₄ est lié au système de cycle par une double liaison et est oxo ; et
R₅ et R₆, indépendamment l'un de l'autre, sont alkyle, alcényle, alcynyle, cycloalkyle, aryle ou hétérocyclyle,
à l'exception de (6S)-6-(tert-butyl-diphényl-silanyloxyméthyl)-3a,4,5,6-tétrahydro-pyrrolo[1,2-b]-isothiazole-1,1-dioxyde, (6S)-(1,1-dioxo-3a,4,5,6-tétrahydro-1H-λ⁶-pyrrolo[1,2-b]isothiazol-6-yl)-méthanol et (3aS)-2,3,3a,4,5,6-hexahydropyrrolo[1,2-b]isothiazole-1,1-dioxyde.

2. Composé selon la revendication 1, **caractérisé en ce qu'**un composé de formule I est un composé de formule

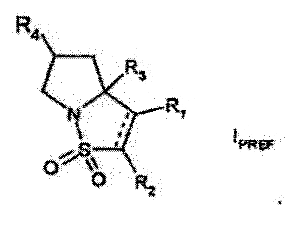

3. Composé selon l'une ou l'autre des revendications 1 et 2, **caractérisé en ce que**
R₁ est (C₁₋₈)alcoxy, ou R₁ est lié au système de cycle par l'intermédiaire d'une double liaison et est oxo,
R₂ est phényle substitué,
R₃ est phénylméthyle substitué, et
R₄ est hydroxy, trialkylsilyloxy, ou R₄ est lié par l'intermédiaire d'une double liaison et est oxo.

4. Composé selon l'une quelconque des revendications 1 à 3, sous forme d'un sel.

5. Composé de formule 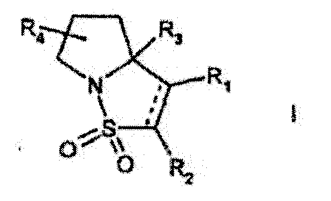 dans laquelle
la ligne en pointillé est une liaison ou n'est pas une liaison,
R₁ est hydrogène, alkyle, alcényle, alcynyle, cycloalkyle, alcoxy, aryle ou hétérocyclyle éventuellement substitué, hydroxy, SH, SR₅, cyano, halogène ou amino, ou
la ligne en pointillé n'est pas une liaison et R₁ est lié au système de cycle par l'intermédiaire d'une double liaison et est oxo,
R₂ est hydrogène ou cycloalkyle, aryle ou hétérocyclyle éventuellement substitué,
R₃ est hydrogène, COOR₆, aminocarbonyle, ou alkyle, alcényle, alcynyle, aralkyle, alcoxy, cycloalkyloxy, aryloxy ou hétérocyclyloxy éventuellement substitué,
R₄ est hydrogène, halogène, hydroxy, SH, alkyle, alcényle, alcynyle, alcoxy ou alkylthio éventuellement substitué, ou un groupement silyle tel que trialkylsilyle ou trialkylsilyloxy, par exemple tri(C₁₋₆)alkylsilyl(oxy), N₃, amino, ou
R₄ est hétérocyclyle comprenant au moins un atome d'azote en tant qu'hétéroatome et étant lié par l'intermédiaire de cet atome d'azote à un composé de formule I, ou
R₄ est lié au système de cycle par une double liaison et est oxo ; et
R₅ et R₆, indépendamment l'un de l'autre, sont alkyle, alcényle, alcynyle, cycloalkyle, aryle ou hétérocyclyle,
ou un sel pharmaceutiquement acceptable de celui-ci, destiné à être utilisé dans le traitement ou la prévention d'un trouble médié par le LFA-1 avec ses ligands impliqués dans l'adhésion, la migration et l'activation cellulaires, ledit trouble étant choisi parmi les affections immunitaires, inflammatoires et allergiques.

6. Composé de formule 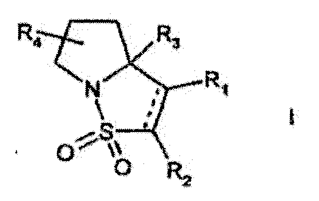 dans laquelle
R₁ est (C₁₋₈)alcoxy, ou R₁ est lié au système de cycle par l'intermédiaire d'une double liaison et est oxo,
R₂ est phényle substitué,
R₃ est phénylméthyle substitué, et
R₄ est hydroxy, trialkylsilyloxy, ou R_{4EX} est lié par l'intermédiaire d'une double liaison et est oxo,
ou un sel pharmaceutiquement acceptable de celui-ci, destiné à être utilisé selon la revendication 5.

7. Utilisation d'un composé de formule 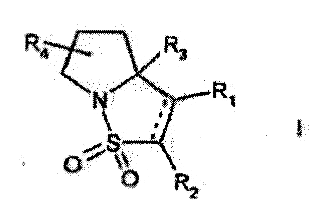 dans laquelle
la ligne en pointillé est une liaison ou n'est pas une liaison,
R₁ est hydrogène, alkyle, alcényle, alcynyle, cycloalkyle, alcoxy, aryle ou hétérocyclyle éventuellement substitué, hydroxy, SH, SR₅, cyano, halogène ou amino, ou
la ligne en pointillé n'est pas une liaison et R₁ est lié au système de cycle par l'intermédiaire d'une double liaison et est oxo,
R₂ est hydrogène ou cycloalkyle, aryle ou hétérocyclyle éventuellement substitué,
R₃ est hydrogène, COOR₆, aminocarbonyle, ou alkyle, alcényle, alcynyle, aralkyle, alcoxy, cycloalkyloxy, aryloxy ou hétérocyclyloxy éventuellement substitué,
R₄ est hydrogène, halogène, hydroxy, SH, alkyle, alcényle, alcynyle, alcoxy ou alkylthio éventuellement substitué, ou un groupement silyle tel que trialkylsilyle ou trialkylsilyloxy, par exemple tri(C₁₋₆)alkylsilyl(oxy), N₃, amino, ou
R₄ est hétérocyclyle comprenant au moins un atome d'azote en tant qu'hétéroatome et étant lié par l'intermédiaire de cet atome d'azote à un composé de formule I, ou
R₄ est lié au système de cycle par une double liaison et est oxo ; et
R₅ et R₆, indépendamment l'un de l'autre, sont alkyle, alcényle, alcynyle, cycloalkyle, aryle ou hétérocyclyle,
ou un sel pharmaceutiquement acceptable de celui-ci, pour la fabrication d'un médicament destiné au traitement des troubles mentionnés dans la revendication 5.
